# EUROPÄISCHE PATENTANMELDUNG

(11) **EP 2 764 918 A1**
(43) Veröffentlichungstag der Anmeldung: **13.08.2014**
(21) Anmeldenummer: 13154868.7
(22) Anmeldetag: 12.02.2013
(51) Int. Cl.: B01L 3/00, A61B 10/00

(54) **Vorrichtung zur Analyse einer Test-Flüssigkeit**

(71) Anmelder: Sulzer Mixpac AG, 9469 Haag (CH)
(72) Erfinder: Ettlin, Josef, 9453 Eichberg (CH)
(74) Vertreter: Manitz, Finsterwald & Partner GbR

(57) **Zusammenfassung**

Die Erfindung betrifft eine Vorrichtung zur Analyse einer Test-Flüssigkeit.

Die erfindungsgemässe Vorrichtung (10) zur Analyse einer Test-Flüssigkeit weist eine Eingangskammer (54), eine Aufbereitungskammer (36, 37) und ein Analyseelement (39) auf. Die Eingangskammer (54) ist dazu vorgesehen, die Test-Flüssigkeit aufzunehmen. Test-Flüssigkeit kann von der Eingangskammer (54) über die Aufbereitungskammer (36, 37) auf das Analyseelement (39) gebracht werden.

Um eine einfache und zuverlässig handhabbare Vorrichtung zu ermöglichen, ist ein Volumen der Aufbereitungskammer (36, 37) vergrösserbar, wobei durch eine Vergrösserung des Volumens der Aufbereitungskammer (36, 37) Test-Flüssigkeit aus der Eingangskammer (54) in die Aufbereitungskammer (36, 37) angesaugt wird. Damit kann eine genau festgelegte Menge Test-Flüssigkeit in die Aufbereitungskammer (36, 37) gesaugt werden, was genaue Analyseergebnisse möglich macht.

## Beschreibung

Die Erfindung betrifft eine Vorrichtung zur Analyse einer Test-Flüssigkeit gemäß dem Oberbegriff des Anspruchs 1.

Derartige Vorrichtungen können beispielsweise zur Analyse von Speichel oder Urin einer Testperson verwendet werden. Es ist aber auch möglich, dass andere Flüssigkeiten analysiert werden oder eine zu analysierende Proben-Substanz beispielsweise in Form eines Feststoffs zunächst mit einer so genannten Auswaschflüssigkeit gemischt oder darin aufgelöst wird und die dabei erhaltene Test-Flüssigkeit anschliessend analysiert wird. Die Analyse kann beispielsweise für den Nachweis von Drogen, Sprengstoffen oder anderer Stoffe wie beispielsweise Spuren von Nüssen verwendet werden.

Bei derartigen Analysen wird meist zunächst die Probe-Substanz, beispielsweise in Form von Speichel mit der Auswaschflüssigkeit gemischt, womit eine Test-Flüssigkeit entsteht, die analysiert werden kann. Die Mischung wird insbesondere so erreicht, dass ein Probensammler, mit der die Probe-Substanz aufgenommen werden kann, in der Auswaschflüssigkeit ausgewaschen wird. Anschliessend wird die Test-Flüssigkeit üblicherweise aufbereitet. Dazu kann die Test-Flüssigkeit mit einem Reaktionspartner beispielsweise in Form von Goldkunjugat in Verbindung gebracht werden. Die Aufbereitung wird auch als eine so genannte Inkubation bezeichnet. Nach einer festgelegten Wartezeit wird die so aufbereitete Test-Flüssigkeit auf ein Analyseelement geleitet, das beispielsweise einen Teststreifen enthält, der auch als ein so genanntes Lateral Flow Assay bezeichnet wird. Der Teststreifen wird an einem Ende mit der Test-Flüssigkeit beaufschlagt, welche dann durch den Teststreifen fliesst und sich in einem Sammelpad am gegenüberliegenden Ende des Teststreifens sammelt. Der Teststreifen kann eine nitrocellulose Membran aufweisen, auf der Testlinien und Kontrolllinien angeordnet sind, die mit der Test-Flüssigkeit reagieren und auf denen eine Reaktion und damit das Analyseergebnis ablesbar sind. Das Analyseergebnis kann beispielsweise mittels einer speziellen elektronischen Auswertevorrichtung abgelesen und verarbeitet werden.

In der US 2006/0292034 A1 wird eine Vorrichtung zur Analyse einer Test-Flüssigkeit beschrieben. Die Vorrichtung verfügt über eine Eingangskammer, eine Aufbereitungskammer und ein Analyseelement mit einem Teststreifen. Die Test-Flüssigkeit in Form von Speichel einer Testperson kann mit einem Probensammler aufgenommen und in die Eingangskammer eingebracht werden. Der Probensammler wird dabei ausgedrückt, so dass Test-Flüssigkeit in die Eingangskammer gelangt. Durch Drücken des Probensammlers auf den Boden der Eingangskammer wird diese in Richtung der darunter angeordneten Aufbereitungskammer verschoben, wodurch ein Volumen der Aufbereitungskammer verkleinert und damit verändert wird. Die Eingangskammer wird so weit verschoben, bis der Boden der Eingangskammer reisst und so eine nicht genau definierte Menge der Test-Flüssigkeit von der Eingangskammer in die Aufbereitungskammer fliesst. Nach einer festgelegten Inkubationszeit von ca. 2 - 3 Minuten wird ein zwischen Aufbereitungskammer und Teststreifen angeordneter Schieber geöffnet, so dass die aufbereitete Test-Flüssigkeit auf ein Ende des Teststreifens gelangen kann. Nach einer Reaktionszeit kann dann wie oben beschrieben das Analyseergebnis auf dem Teststreifen abgelesen werden.

In der DE 20 2008 017 883 U1 ist ebenfalls eine Vorrichtung zur Analyse einer Test-Flüssigkeit beschrieben. Die Vorrichtung verfügt über eine Aufbereitungskammer in Form einer Mischkammer und ein Analyseelement mit einem Teststreifen. Bei der Nutzung der Vorrichtung wird Test-Flüssigkeit aus einer separaten Auswaschvorrichtung in die Aufbereitungskammer gefüllt, von der sie nach der Aufbereitung auf den Teststreifen geleitet wird. Damit eine bestimmte Menge an Test-Flüssigkeit in die Aufbereitungskammer eingefüllt wird, weist sie eine Füllstandsmarkierung auf, bis zu der ein Anwender der Vorrichtung Test-Flüssigkeit einfüllen soll. Eine Überwachung oder Überprüfung der tatsächlich eingefüllten Menge an Test-Flüssigkeit ist nicht möglich.

Demgegenüber ist es die Aufgabe der Erfindung, eine Vorrichtung zur Analyse einer Test-Flüssigkeit vorzuschlagen, welche einfach und zuverlässig handhabbar ist. Erfindungsgemäß wird diese Aufgabe mit einer Vorrichtung zur Analyse einer Test-Flüssigkeit mit den Merkmalen des Anspruchs 1 gelöst.

Die erfindungsgemässe Vorrichtung zur Analyse einer Test-Flüssigkeit weist eine Eingangskammer, eine Aufbereitungskammer und ein Analyseelement auf. Die Eingangskammer ist dazu vorgesehen, die Test-Flüssigkeit aufzunehmen. Test-Flüssigkeit kann von der Eingangskammer über die Aufbereitungskammer auf das Analyseelement gebracht werden und ein Volumen der Aufbereitungskammer ist veränderbar.

Erfindungsgemäss ist das Volumen der Aufbereitungskammer vergrösserbar, wobei durch eine Vergrösserung des Volumens der Aufbereitungskammer Test-Flüssigkeit aus der Eingangskammer in die Aufbereitungskammer angesaugt wird. Bei der Vergrösserung des Volumens der Aufbereitungskammer wird in der Aufbereitungskammer ein Unterdruck erzeugt, auf Grund dessen eine genau festgelegte Menge Test-Flüssigkeit in die Aufbereitungskammer gesaugt wird. Die angesaugte Menge entspricht dabei der Volumenänderung der Aufbereitungskammer. Damit kann einfach sichergestellt werden, dass eine definierte Menge von Test-Flüssigkeit in die Aufbereitungskammer gelangt, was eine definierte Aufbereitung und damit ein genaues Analyseergebnis gewährleistet. Der Anwender der Vorrichtung wird insbesondere dabei unterstützt, das Volumen der Aufbereitungskammer genau um ein definiertes Mass zu vergrössern. Dazu kann beispielsweise ein mechanischer Anschlag, der die Volumenvergrösserung begrenzt, oder eine Rastierung oder Fixierung einer Ausgangsposition und einer Aufbereitungsposition eines Betätigungselements, mittels welchem die Volumenvergrösserung durchgeführt werden kann, vorgesehen sein.

Das Analyseelement weist insbesondere einen oder mehrere Teststreifen auf.

In Ausgestaltung der Erfindung ist in einem Ausgangszustand der Vorrichtung in der Eingangskammer eine Auswaschflüssigkeit angeordnet, welche gemischt mit einer Proben-Substanz die Test-Flüssigkeit ergibt. Unter einem Ausgangszustand der Vorrichtung soll ein unbenutzter Zustand, also der Zustand vor dem Beginn einer Analyse einer Proben-Substanz oder einer Test-Flüssigkeit verstanden werden. Die Proben-Substanz kann beispielsweise als eine Flüssigkeit, ein Feststoff oder eine beliebige Mischung aus Flüssigkeit und Feststoff ausgeführt sein. Beispiele für Probe-Substanzen sind Speichel, Urin, Blut, Abstriche von Gegenständen oder Lebensmittelproben. Durch das Bereitstellen von Auswaschflüssigkeit in der Eingangskammer kann auf einem separaten Behälter für die Auswaschflüssigkeit verzichtet werden, der gesondert verpackt und mit ausgeliefert werden müsste. Ausserdem ist die Handhabung für den Anwender besonders einfach, da er nicht mit mehreren Behältern hantieren muss.

Die erfindungsgemässe Vorrichtung kann einen Deckel aufweisen, mittels welchem die Eingangskammer verschlossen werden kann. Es ist auch möglich, dass im Ausgangszustand ein Probensammler zumindest teilweise in der Eingangskammer angeordnet ist und dieser die Eingangskammer so abschliesst, dass keine Auswaschflüssigkeit austreten kann. Es ist auch möglich, dass eine Hülle für den Probensammler vorgesehen ist, in der der Probensammler im Ausgangszustand aufbewahrt wird. Die genannte Hülle ist dann insbesondere so ausgeführt, dass sie teilweise in der Eingangskammer angeordnet werden kann und sie damit verschliesst. Die Hülle kann dann auch nach dem Auswaschen des Probensammlers dazu dienen, die Eingangskammer zu verschliessen und so ein Austreten von Auswasch- oder Test-Flüssigkeit zu verhindern.

In Ausgestaltung der Erfindung weist die Vorrichtung ein Gehäuse mit einem Gehäuse-Innenraum auf. Teilweise oder komplett innerhalb des Gehäuse-Innenraums ist ein Aufbereitungselement angeordnet, welches die Aufbereitungskammer teilweise oder vollständig begrenzt. Das Aufbereitungselement kann auch zwei separate Aufbereitungskammern begrenzen. Ebenfalls teilweise oder vollständig innerhalb des Gehäuse-Innenraums ist ein Eingangselement angeordnet, welches die Eingangskammer teilweise oder vollständig begrenzt. Das Aufbereitungselement und das Eingangselement sind in einer Axialrichtung hintereinander angeordnet und so miteinander verbunden, dass eine Verschiebung des Eingangselements in Axialrichtung vom Aufbereitungselement weg das Volumen der Aufbereitungskammer vergrössert. Damit wird eine kompakte Ausführung der Vorrichtung ermöglicht. Da die Eingangskammer sowieso von ausserhalb des Gehäuses zugänglich sein muss, um die Proben-Substanz einzubringen, kann durch die beschriebene Ausführung der Vorrichtung das Volumen der Aufbereitungskammer vergrössert werden, ohne dass sie selbst von ausserhalb des Gehäuses zugänglich sein muss.

Das Eingangselement und das Aufbereitungselement weisen insbesondere jeweils eine hohlzylindrische Grundform auf, so dass die genannte Axialrichtung der Axialrichtung des Eingangselements und des Aufbereitungselements entspricht. Durchmesser des Eingangselements und des Aufbereitungselements weichen insbesondere voneinander, so dass sich der korrespondierende Gehäuse-Innenraum hauptsächlich aus Zylindern mit unterschiedlichen Durchmessern zusammensetzt. Insbesondere ist der Durchmesser des Aufbereitungselements grösser als der Durchmesser des Eingangselements, so dass das Gehäuse einen Anschlag aufweist, der die axiale Ausdehnung des Aufbereitungselements und damit die Vergrösserung des Volumens der Aufbereitungskammer begrenzt und somit die Vergrösserung des Volumens der Aufbereitungskammer um ein definiertes Mass ermöglicht wird.

Die Kopplung des Aufbereitungselements mit dem Eingangselement erfolgt insbesondere über eine umlaufende, nach innen gerichtete Nut des Aufbereitungselements und einen korrespondierenden Bund des Eingangselements. Die Anordnung von Nut und Bund kann auch umgekehrt sein.

Das Aufbereitungselement kann insbesondere über umlaufende Falten verfügen, die zur Vergrösserung des Volumens der Aufbereitungskammer auseinandergezogen werden und so die Volumenvergrösserung erlauben. Alternativ oder zusätzlich kann das Aufbereitungselement aus einem elastischen Material wie beispielsweise einem Elastomer oder Silikon bestehen.

Damit bei einer Verschiebung des Eingangselements in eine von der Aufbereitungskammer weg gerichtete Richtung nicht das Aufbereitungselement komplett mit verschoben wird, ist eine Fixierung des Eingangselements in Axialrichtung gegenüber dem Gehäuse notwendig. Diese kann beispielsweise durch einen umlaufenden Bund an einem dem Eingangselement entgegen liegenden Ende des Aufbereitungselements, das an einer korrespondierenden Kante des Gehäuses anliegt, realisiert werden.

In Ausgestaltung der Erfindung weist das Eingangselement ein Betätigungselement auf, das ausserhalb des Gehäuses angeordnet ist. Das Betätigungselement ist so ausgeführt ist, dass über das Betätigungselement eine Kraft zum Verschieben des Eingangselements einleitbar ist. Damit kann auf einfache Weise das Eingangselement verschoben und so Test-Flüssigkeit in die Aufbereitungskammer angesaugt werden.

Das Eingangselement und das Betätigungselement sind insbesondere einstückig ausgeführt. Es ist aber auch möglich, dass sie als separate Teile ausgeführt sind, die geeignet miteinander verbunden sind. Das Betätigungselement weist insbesondere die gleiche Aussenkontur wie das Gehäuse auf und schliesst sich in Axialrichtung an das Gehäuse an.

Das Betätigungselement kann zum Verschieben des Eingangselements gegenüber dem Gehäuse in Axialrichtung verschoben werden oder insbesondere gedreht werden. Zur Umsetzung der Drehbewegung in eine Axialbewegung, also ein Verschieben des Eingangselements, weist das Eingangselement ein erstes Gewinde und das Gehäuse ein korrespondierendes zweites Gewinde auf. Damit ist eine besonders einfache Handhabung der Vorrichtung möglich.

Dabei kann das Eingangselement ein Aussengewinde und das Gehäuse ein Innengewinde aufweisen oder umgekehrt. Die Vorrichtung kann beispielsweise so ausgelegt sein, dass eine Drehung des Betätigungselements gegenüber dem Gehäuse um 180° das Eingangselement so weit verschiebt, dass das Aufbereitungselement an dem Anschlag des Gehäuses ansteht und so die vorgesehene Menge an Test-Flüssigkeit aus der Eingangskammer in die Aufbereitungskammer angesaugt wird.

In Ausgestaltung der Erfindung kann das Eingangselement zur Vergrösserung des Volumens der Aufbereitungskammer gegenüber dem Gehäuse von einer Ausgangsposition in eine Aufbereitungsposition verdreht werden. Eine Verbindung zwischen der Eingangskammer und der Aufbereitungskammer besteht nur zwischen der Ausgangsposition und der Aufbereitungsposition. Damit besteht in den beiden genannten Positionen keine Verbindung zwischen der Eingangskammer und der Aufbereitungskammer. Damit wird zum einen verhindert, dass in der Ausgangsposition ungewollt Test-Flüssigkeit in die Aufbereitungskammer gelangt und zum anderen wird verhindert, dass während oder nach der Aufbereitung noch weitere Test-Flüssigkeit nach fliessen und so das Analyseergebnis beeinflussen kann. Damit werden sehr exakte Analysen möglich.

Dazu weist beispielsweise das Eingangselement ein oder mehrere Durchgänge in Richtung Aufbereitungselement auf. Das Aufbereitungselement weist korrespondierende Durchgänge oder Löcher auf, die so angeordnet sind, dass sie sich nur zwischen der Ausgangsposition und der Aufbereitungsposition mit den Durchgängen des Eingangselements überdecken.

In Ausgestaltung der Erfindung ist zwischen der Aufbereitungskammer und dem Analyseelement eine Folie angeordnet, welche in unversehrtem Zustand die Aufbereitungskammer vom Analyseelement abtrennt. Zum Starten der Analyse der Test-Flüssigkeit kann die Folie mittels eines Dorns durchstochen werden, so dass aufbereitete Test-Flüssigkeit von der Aufbereitungskammer zum Analyseelement fliessen kann. Damit werden ein einfacher und kostengünstiger Aufbau der Vorrichtung und eine einfache Handhabung ermöglicht.

Insbesondere weist das Analyseelement den genannten Dorn auf und ist schwenkbar mit dem Gehäuse verbunden. Es kann gegenüber dem Gehäuse eine Ruheposition und eine Anzeigeposition einnehmen, wobei es so ausgeführt und angeordnet ist, dass bei einem Verschwenken gegenüber dem Gehäuse der Dorn des Analyseelements die zwischen Aufbereitungskammer und dem Analyseelement angeordnet Folie durchsticht und so die Analyse der Test-Flüssigkeit gestartet wird. Damit kann der Analysevorgang sehr einfach durch Verschwenken des Analyseelements gestartet werden.

Das Analyseelement weist dabei insbesondere eine hauptsächlich quaderförmige Grundform auf. In der Ruheposition ist eine Haupterstreckungsrichtung des Analyseelements hauptsächlich parallel zur Axialrichtung angeordnet. Um es in die Anzeigeposition zu bringen, wird es beispielsweise um 90° gegenüber dem Gehäuse verschwenkt beziehungsweise ausgeklappt. Das Analyseelement enthält meist einen oder mehrere Teststreifen, an deren Form es angepasst ist. In diesem Fall verläuft die genannte Haupterstreckungsrichtung in Fliessrichtung des Teststreifens. Das Analyseelement ist im Ausgangszustand der Vorrichtung in der beschriebenen Ruheposition. Damit ist die Vorrichtung im Ausgangszustand, in der es auch gelagert und verkauft wird, sehr kompakt und damit einfach und kostengünstig zu lagern und zu transportieren. Das Analyseelement ist insbesondere so angeordnet, dass Öffnungen zum Ablesen des Analyseergebnisses in der Ruheposition zum Gehäuse hin orientiert und damit geschützt sind.

Die schwenkbare Anordnung des Analyseelements gegenüber dem Gehäuse ist unabhängig von der Art, wie Test-Flüssigkeit von der Eingangskammer in die Aufbereitungskammer gebracht wird. Die beschriebene Anordnung kann damit auch mit jeder anderen möglichen Art Test-Flüssigkeit von der Eingangskammer in die Aufbereitungskammer zu bringen, kombiniert werden.

In Ausgestaltung der Erfindung verfügt die Vorrichtung über ein Gehäuse, welches die Eingangskammer, einen Gehäuse-Innenraum, eine Zulaufleitung von der Eingangskammer zum Gehäuse-Innenraum und eine Ablaufleitung vom Gehäuse-Innenraum zum Analyseelement aufweist. Im Gehäuse-Innenraum ist ein Stössel mit einem Absatz verschieblich angeordnet. Ausserdem ist auf dem Stössel ein Ringkolben angeordnet, der sowohl im Gehäuse-Innenraum, als auch gegenüber dem Stössel zumindest stückweise verschoben werden kann. Die Aufbereitungskammer wird in diesem Fall von einer dem Ringkolben zugewandten Stirnfläche des genannten Absatzes des Stössels, dem Gehäuse-Innenraum und dem Ringkolben gebildet. Der Stössel und der Ringkolben sind dabei so ausgeführt und angeordnet, dass bei einer Verschiebung des Stössels von einer Ausgangsposition in eine Aufbereitungsposition der Ringkolben zunächst bezogen auf den Gehäuse-Innenraum stehen bleibt und so das Volumen der Aufbereitungskammer vergrössert wird. Dabei ist es nicht notwendig, dass der Ringkolben sich überhaupt nicht bewegt. Eine kleine Verschiebung des Ringkolbens ist zulässig, sie muss aber deutlich kleiner sein als die Verschiebung des Stössels. Dies ermöglicht einen einfachen Aufbau der Vorrichtung.

Damit der Ringkolben zunächst stehen bleibt, können beispielsweise die einzelnen Teile so ausgeführt sein, dass die Reibung zwischen Ringkolben und Gehäuse-Innenraum grösser ist als die Reibung zwischen Ringkolben und Stössel. Zusätzlich oder alternativ kann der Gehäuse-Innenraum einen Absatz aufweisen, der den Ringkolben zunächst in seiner Position hält.

Der Gehäuse-Innenraum weist insbesondere eine hauptsächlich zylinderförmige Form auf. Seine Achse verläuft dabei insbesondere parallel zur Haupterstreckungsrichtung des Analyseelements. Dies ermöglicht einen besonders kompakten Aufbau der Vorrichtung.

In Ausgestaltung der Erfindung sind der Stössel und der Ringkolben so ausgeführt und angeordnet, dass in der Ausgangsposition die Zulaufleitung von der Eingangskammer zum Gehäuse-Innenraum verschlossen ist. Damit besteht in der Ausgangsposition keine Verbindung zwischen der Eingangskammer und der Aufbereitungskammer. Damit wird verhindert, dass in der Ausgangsposition ungewollt Test-Flüssigkeit in die Aufbereitungskammer gelangt. Die Zulaufleitung wird dabei insbesondere vom Stössel verschlossen, der dazu eine entsprechende Form aufweist und zusätzlich über Dichtelemente, wie beispielsweise O-Ringe verfügen kann.

Insbesondere ist in der Aufbereitungsposition die Ablaufleitung vom Gehäuse-Innenraum zum Analyseelement verschlossen. Damit wird verhindert, dass die Test-Flüssigkeit vor Abschluss der Aufbereitung bereits zum Analyseelement gelangt. Dazu wird insbesondere auch der Stössel eingesetzt, wobei die genannten oder zusätzliche Dichtelemente ebenfalls wirken können.

In Ausgestaltung der Erfindung weist der Stössel einen Mitnehmer auf, welcher so ausgeführt ist, dass er eine Relativbewegung zwischen Stössel und Ringkolben begrenzt. Der Mitnehmer ist beispielsweise als ein Bereich mit einem grösseren Durchmesser als der Durchgang des Ringkolbens ausgeführt. Der Stössel und der Ringkolben sind in der Aufbereitungsposition so angeordnet, dass die Zulaufleitung von der Eingangskammer zum Gehäuse-Innenraum verschlossen ist. Damit wird verhindert, dass während oder nach der Aufbereitung noch weitere Test-Flüssigkeit nach fliessen und so das Analyseergebnis beeinflussen kann. Dies wird insbesondere so umgesetzt, dass der Ringkolben ausgehend von seiner Position in der Ausgangsposition vom Mitnehmer so weit mitgenommen wird, dass er die Zulaufleitung verschliesst. Zur besseren Abdichtung kann der Ringkolben über umlaufende Dichtlippen oder über zusätzliche Dichtelemente, beispielsweise in Form von O-Ringen verfügen.

Ausserdem können der Stössel und der Ringkolben eine Analyseposition einnehmen, in welcher die Ablaufleitung vom Gehäuse-Innenraum zum Analyseelement geöffnet und eine Verbindung von der Eingangskammer zur Aufbereitungskammer unterbrochen ist. Damit wird ermöglicht, dass aufbereitete Test-Flüssigkeit zum Analyseelement gelangen kann und gleichzeitig verhindert, dass dabei noch weitere, nicht aufbereitete Test-Flüssigkeit in die Aufbereitungskammer fliesst. Die genannte Verbindung von der Eingangskammer zur Aufbereitungskammer wird dabei insbesondere vom Ringkolben und dem Stössel unterbrochen. Es kann dabei möglich sein, dass die Zulaufleitung von der Eingangskammer zum Gehäuse-Innenraum zwar offen ist, aber keine Test-Flüssigkeit von der Zulaufleitung in die Aufbereitungskammer gelangen kann.

In Ausgestaltung der Erfindung weist die Vorrichtung ein gegenüber dem Gehäuse verschieblich angeordnetes und mit dem Stössel gekoppeltes Betätigungselement auf, mittels welchem eine Kraft zum Verschieben des Stössels eingeleitet werden kann. Damit wird eine einfache Handhabung der Vorrichtung ermöglicht. Es ist möglich, dass zur sicheren Einstellung der verschiedenen Positionen Rastierungen zwischen Gehäuse und Betätigungselement vorgesehen sind, die das Betätigungselement in den einzelnen Positionen fixieren, aber überdrückt werden können. Dazu kann das Betätigungselement eine oder mehrere Ausnehmungen und das Gehäuse entsprechend positionierte Erhebungen aufweisen.

Das Betätigungselement kann insbesondere eine oder mehrere Ausnehmungen aufweisen, durch welche Markierungen, welche eine aktuelle Position des Eingangselements oder des Stössels anzeigen, sichtbar sind. Ausserdem kann auch über entsprechende Wartezeiten in den einzelnen Positionen informiert werden. Damit kann ein Anwender einfach über den aktuellen Fortschritt der Analyse informiert und auf eventuell einzuhaltende Wartezeiten hingewiesen werden. Damit wird eine sichere Handhabung und damit auch zuverlässige Analyseergebnisse erreicht.

In Ausgestaltung der Erfindung weist das Gehäuse einen Aufbewahrungsraum für einen Probensammler auf. Damit können alle für die Analyse der Test-Substanz notwendigen Teile zusammengefasst werden, was eine einfache Handhabung ermöglicht. Wie bereits ausgeführt kann die Eingangskammer als Aufbewahrungsraum dienen. Es ist aber auch möglich, dass ein zusätzlicher Aufbewahrungsraum vorgesehen ist.

Die einzelnen Teile der Vorrichtung können beispielsweise aus Polyethylen oder Polypropylen bestehen und insbesondere mittels eines Spritzgussverfahrens hergestellt sein.

Weitere Vorteile, Merkmale und Einzelheiten der Erfindung ergeben sich anhand der nachfolgenden Beschreibung von Ausführungsbeispielen sowie anhand der Zeichnungen, in welchen gleiche oder funktionsgleiche Elemente mit identischen Bezugszeichen versehen sind.

Dabei zeigen:
- Fig. 1: eine Vorrichtung zur Analyse einer Test-Flüssigkeit in einem Ausgangszustand,
- Fig. 2: die Vorrichtung aus Fig. 1 in einer Analyseposition,
- Fig. 3: ein Aufbereitungselement der Vorrichtung aus Fig. 1,
- Fig. 4: eine alternative Ausführungsform einer Vorrichtung zur Analyse einer Test-Flüssigkeit in einem Ausgangszustand,
- Fig. 5: eine weitere alternative Ausführungsform einer Vorrichtung zur Analyse einer Test-Flüssigkeit in einer Analyseposition,
- Fig. 6: die Vorrichtung aus Fig. 5 in einer Ausgangsposition mit teilweise frei geschnittenen Details,
- Fig. 7: eine Prinzipdarstellung von Einzelteilen der Vorrichtung aus Fig. 5 in einer Ausgangsposition
- Fig. 8: die Einzelteile aus Fig. 7 in einer Aufbereitungsposition und
- Fig. 9: die Einzelteile aus Fig. 7 und 8 in der Analyseposition.

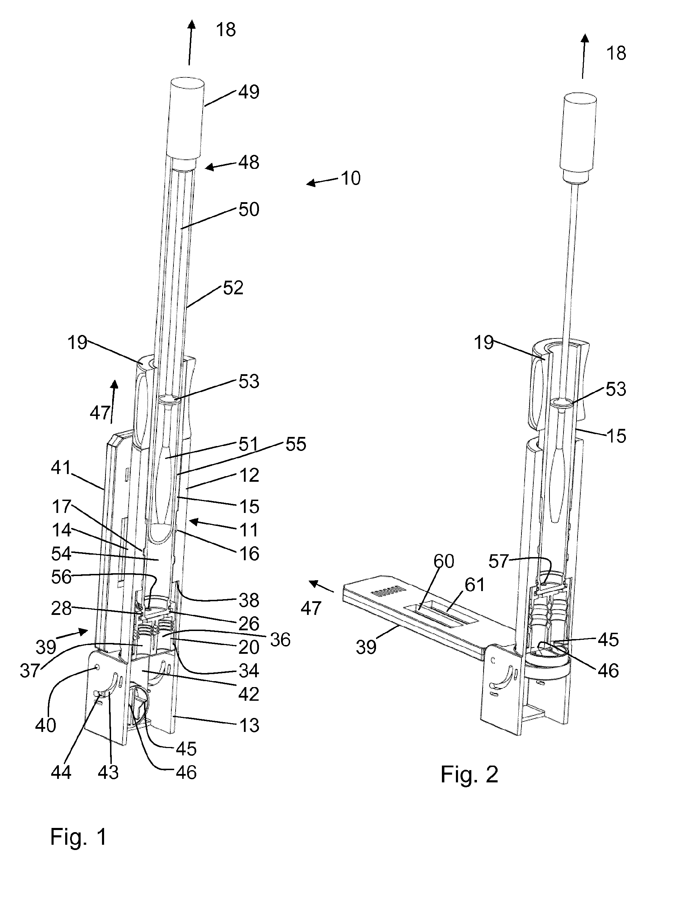

Gemäss Fig. 1 weist eine Vorrichtung 10 zur Analyse einer Test-Flüssigkeit ein Gehäuse 11 auf, das sich aus einem hauptsächlich zylinderförmigen Abschnitt 12 und einem, sich nach unten anschliessenden quaderförmigen Abschnitt 13 zusammen setzt. Der zylinderförmige Abschnitt 12 des Gehäuses 11 bildet dabei einen Gehäuse-Innenraum 55 aus. Der quaderförmige Abschnitt 13 ist so ausgeführt, dass man die Vorrichtung 10 auf einer ebenen Fläche so abstellen kann, dass der zylinderförmige Abschnitt 12 senkrecht nach oben zeigt. Innerhalb des zylinderförmigen Abschnitts12 des Gehäuses 11 ist in einem oberen Bereich 14 ein hauptsächlich zylinderförmiges Eingangselement 15 so angeordnet, dass ein Teil des Eingangselements 15 aus dem zylinderförmigen Abschnitt 12 des Gehäuses 11 heraus ragt. Das Eingangselement 15 begrenzt eine Eingangskammer 54. Der zylinderförmige Abschnitt 12 des Gehäuses 11 weist ein Innengewinde 16 und das Eingangselement 15 ein korrespondierendes Aussengewinde 17 auf. Durch Verdrehen des Eingangselements 15 gegenüber dem zylinderförmigen Abschnitt 12 des Gehäuses 11 kann damit das Eingangselement 15 in Axialrichtung 18 des zylinderförmigen Abschnitts 12 des Gehäuses 11 weiter aus dem zylinderförmigen Abschnitt 12 des Gehäuses 11 heraus geschoben werden. Um eine Kraft zum Drehen und damit zum Verschieben des Eingangselements 15 einleiten zu können, ist an dem Teil des Einganselements 15, welcher aus dem zylinderförmigen Abschnitt 12 des Gehäuses 11 herausragt, ein Betätigungselement 19 drehfest angeordnet. Das Betätigungselement 19 weist eine hohlzylindrische Grundform auf, welche sich an die Form des zylinderförmigen Abschnitts 12 des Gehäuses 11 anpasst. Um das Betätigungselement 19 besser greifen zu können, weist es an seinem Umfang verteilt insgesamt vier Einbuchtungen auf.

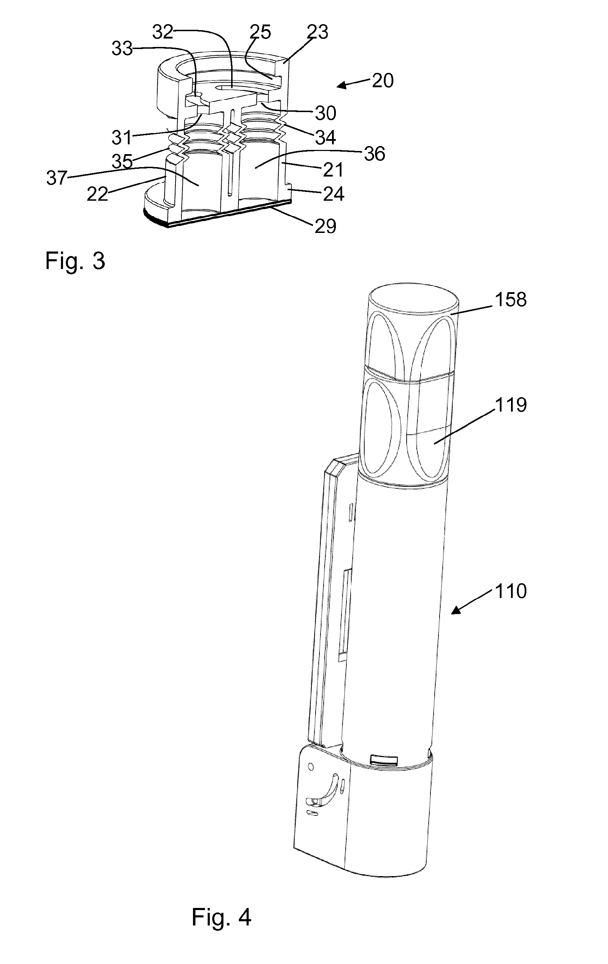

An das Eingangselement 15 schliesst sich in Richtung quaderförmigem Abschnitt 13 des Gehäuses 11 ein Aufbereitungselement 20 an, das in Fig. 3 detaillierter dargestellt ist. Das Aufbereitungselement 20 weist zwei hauptsächlich zylindrischen Mittelteile 21, 22 auf, die parallel zueinander und koaxial zur Axialrichtung 18 angeordnet sind. Die Mittelteile 21, 22 weisen jeweils umlaufende Falten 34, 35 auf, die auseinander gezogen werden können. Die Mittelteile 21, 22 werden über einen Anschlussbund 23 in Richtung Betätigungselement 19 und in Richtung quaderförmigem Abschnitt 13 des Gehäuses 11 über einen Abschlussbund 24 verbunden. Der Anschlussbund 23 weist eine zylindrische Aussenkontur auf, die an die Innenkontur des zylinderförmigen Abschnitts 12 des Gehäuses 11 angepasst ist. Er weist ausserdem eine innen umlaufende Nut 25 auf, in die ein korrespondierender Bund 26 des Eingangselements 15 eingreift, so dass bei einer Verschiebung des Eingangselement 15 in Axialrichtung 18 der Anschlussbund 23 des Eingangselements 20 mitgenommen wird. Zwischen dem Eingangselement 15 und dem Anschlussbund 23 des Aufbereitungselements 20 ist ausserdem ein Dichtelement 28 in Form eines O-Rings angeordnet. Der Abschlussbund 24 weist ebenfalls eine zylindrische Aussenkontur auf, die an die Innenkontur des zylinderförmigen Abschnitts 12 des Gehäuses 11 angepasst ist. Er ist in Richtung des quaderförmigen Abschnitts13 des Gehäuses 11 mit einer Folie 29 abgeschlossen. Im eingebauten Zustand liegt der Abschlussbund 24 an einer Kante 34 des zylinderförmigen Abschnitts 12 des Gehäuses 11 an, so dass er nicht in Richtung Betätigungselement 19 verschoben werden kann. Damit wird bei einer Verschiebung des Eingangselements 15 in Axialrichtung 18 nur der Anschlussbund 23 des Aufbereitungselements 20 mitgenommen und der Abschlussbund 24 bleibt stehen. Dabei werden die Falten 34, 35 der Mittelteile 21, 22 auseinander gezogen. Der zylinderförmige Abschnitt 12 des Gehäuses 11 weist einen Anschlag 38 auf, an den der Anschlussbund 24 nach einem definierten Verschiebeweg anschlägt, womit die Verschiebung des Anschlussbunds 24 begrenzt wird. Die Gewinde 16, 17, der Anschlag 38 und die Einbauposition des Aufbereitungselements 20 sind so aufeinander abgestimmt, dass ausgehend von einer Ausgangsposition des Betätigungselements 19 und damit des Eingangselements 15 der Anschlussbund 23 nach einer Drehung des Betätigungselements 19 gegenüber dem Gehäuse 11 um 180° am Anschlag 38 anschlägt, womit eine Aufbereitungsposition erreicht ist.

Die Mittelteile 21, 22 sind in Richtung Anschlussbund 23 bis auf jeweils ein kreisrundes Loch 30, 31 abgeschlossen. Damit bilden die Mittelteile 21, 22 des Aufbereitungselements 20 zwei voneinander getrennte Aufbereitungskammern 36, 37 aus, deren Volumen wie beschrieben um ein definiertes Mass vergrössert werden kann. Die Löcher 30, 31 sind mit jeweils kreisbogen-förmigen Zuläufen 32, 33 verbunden, die in Richtung Anschlussbund 23 offen und in Richtung Abschlussbund 24 geschlossen sind. Der Zweck der Zuläufe 32, 33 wird weiter unten näher erläutert.

Am quaderförmigen Abschnitt 13 des Gehäuses 11 ist schwenkbar ein Analyseelement 39 angeordnet. Das Analyseelement 39 weist eine hauptsächlich quaderförmige Grundform mit einer Haupterstreckungsrichtung 47 auf. Es enthält zwei nicht näher dargestellte Teststreifen, die so angeordnet sind, dass ihre Fliessrichtung in Richtung der Haupterstreckungsrichtung 47 verläuft. Die Teststreifen sind über zwei Ausnehmungen 60, 61 des Analyseelements 39 teilweise sichtbar.

Im in Fig. 1 dargestellten Ausgangszustand der Vorrichtung 10 und damit in einer Ruheposition des Analyseelements 39 ist das Analyseelement 39 parallel zur Axialrichtung 18 angeordnet, so dass die Haupterstreckungsrichtung 47 parallel zur Axialrichtung 18 verläuft. Die Ausnehmungen 60, 61, über die die Teststreifen sichtbar sind, sind in diesem Zustand zum Gehäuse 11 hin orientiert. Das Analyseelement 39 ist um eine Achse 40 schwenkbar, wobei ein erster Teil 41 des Analyseelements 39 vom zylinderförmigen Abschnitt 12 des Gehäuses 10 weg schwenkt und ein zweiter Teil 42 in den quaderförmigen Abschnitt 13 des Gehäuses 11 hinein schwenkt. Zur Führung der Schwenkbewegung weist der quaderförmige Abschnitt 13 des Gehäuses 11 eine bogenförmige Ausnehmung 43 auf, in der ein Stift 44 des zweiten Teils 42 des Analyseelements 39 geführt wird. Das Analyseelement 39 kann so weit verschwenkt werden, bis seine Haupterstreckungsrichtung 47 senkrecht zur Axialrichtung 18 ausgerichtet ist. Damit ist eine Anzeigeposition des Analyseelements 39 erreicht, die in Fig. 2 dargestellt ist.

Der zweite Teil 42 des Analyseelements 39 weist zwei Dorne 45, 46 auf, die in Richtung Folie 29 des Aufbereitungselements 20 orientiert sind. Die Dorne 45, 46 sind so ausgeführt, dass sie beim Verschwenken des Analyseelements 39 in die Anzeigeposition die Folie 29 durchstechen und so die Aufbereitungskammern 36, 37 des Aufbereitungselements 20 in Richtung Analyseelement 39 öffnen.

In das Eingangselement 15 ist im in Fig. 1 dargestellten Ausgangszustand ein Probensammler 48 eingesteckt, der über einen Griff 49, einen Schaft 50 und eine Aufnahmespitze 51 verfügt. Der Probensammler 48 ist dabei in eine Hülle 52 so weit eingesteckt, dass nur ein Teil des Griffs 49 aus der Hülle 52 heraus ragt. Die Hülle 52 weist eine hauptsächlich zylinderförmige Aussenkontur auf und ist so dimensioniert, dass sie das Eingangselement 15 in Axialrichtung 18 dicht abschliesst. Der Schaft 50 des Probensammlers 48 weist ausserdem eine Dichtscheibe 53 auf, die in etwa dieselbe Aussenkontur wie die Hülle 52 aufweist. Sie dient zum Abschliessen des Eingangselements 15, wenn der Probensammler 48 wie in Fig. 2 dargestellt ohne Hülle 52 eingesteckt ist. Die Eingangskammer 54 dient in diesem Fall auch als ein Aufbewahrungsraum für den Probensammler 48.

Zur Analyse einer Proben-Substanz, beispielsweise in Form von Speichel einer Testperson, wird ausgehend vom Ausgangszustand der Vorrichtung 10 in Fig. 1 zunächst der Probendsammler 48 inklusive der Hülle 52 aus dem Eingangselement 15 entfernt. Anschliessend wird die Proben-Substanz mit der Aufnahmespitze 51 des Probensammlers 48 aufgenommen und der Probensammler 48 ohne Hülle 52 in das Aufnahmeelement 15 eingesteckt. Damit taucht die Aufnahmespitze 51 in eine sich in der Eingangskammer 54 befindende Auswaschflüssigkeit ein. Durch Schütteln der Vorrichtung 10 wird die aufgenommene Proben-Substanz ausgewaschen. Die Mischung aus Proben-Substanz und Auswaschflüssigkeit ergibt eine Test-Flüssigkeit, die anschliessend analysiert werden kann.

Es ist auch möglich, dass zunächst nur der Probensammler 48 aus der Hülle 52 entfernt wird und die Hülle 52 weiterhin das Eingangselement 15 abschliesst. Erst nach Aufnahme der Test-Substanz wird dann die Hülle 52 entfernt.

Nach dem Auswaschen der Test-Substanz wird jeweils eine definierte Menge an Test-Flüssigkeit aus der Eingangskammer 54 in die beiden Aufbereitungskammern 36, 37 gebracht. Dazu wird das Betätigungselement 19 und damit das Eingangselement 15 gegenüber dem zylinderförmigen Abschnitt 12 des Gehäuses 11 um 180° gedreht und damit das Eingangselement 15 in Axialrichtung 18 verschoben. Die Drehung und die Axialverschiebung werden beendet, wenn der Anschlussbund 23 des Aufbereitungselements 20 an den Anschlag 38 des zylinderförmigen Abschnitts 12 des Gehäuses 11 anschlägt. Damit vergrössern sich die Volumen der Aufbereitungskammern 36, 37 um jeweils ein definiertes Mass. Durch diese Volumenvergrösserung entsteht in den Aufbereitungskammern 36, 37 jeweils ein Unterdruck, auf Grund dessen Test-Flüssigkeit aus der Eingangskammer 54 angesaugt wird. Dabei wird jeweils genau so viel Test-Flüssigkeit angesaugt, wie die Volumen der Aufbereitungskammern 36, 37 vergrössert werden. Die nach der beschriebenen Drehung erreichte Aufbereitungsposition des Eingangselements 15 und damit auch des Aufbereitungselements 20 ist in Fig. 2 dargestellt.

Damit Test-Flüssigkeit von der Eingangskammer 54 in die Aufbereitungskammern 36, 37 gebracht werden kann, weist die Eingangskammer 54 und damit das Eingangselement 15 in Richtung Aufbereitungskammern 36, 37 zwei Durchgänge 56, 57 auf, wobei in Fig. 1 nur der Durchgang 56 und in Fig. 2 nur der Durchgang 57 zu sehen ist. In der in Fig. 1 dargestellten Ausgangsposition und der in Fig. 2 dargestellten Aufbereitungsposition des Eingangselements 15 überdecken sich die Durchgänge 56, 57 nicht mit den kreisbogen-förmigen Zuläufen 32, 33 der Löcher 30, 31 der Mittelteile 21, 22 des Aufbereitungselements 20. Damit ist in diesen Positionen die Eingangskammer 54 von den Aufbereitungskammern 36, 37 getrennt und es kann keine Test-Flüssigkeit von der Eingangskammer 54 in die Aufbereitungskammern 36, 37 strömen. Während der Drehung des Eingangselements 15 von der Ausgangsposition in die Aufbereitungsposition überdecken sich die Durchgänge 56, 57 mit den kreisbogen-förmigen Zuläufen 32, 33 der Löcher 30, 31 der Mittelteile 21, 22 des Aufbereitungselements 20, so dass dann eine Verbindung zwischen der Eingangskammer 54 und der Aufbereitungskammern 36, 37 besteht und Test-Flüssigkeit von der Eingangskammer 54 in die Aufbereitungskammern 36, 37 angesaugt werden kann.

In den Aufbereitungskammern 36, 37 befindet sich jeweils ein Reaktionspartner, beispielsweise in Form von Goldkunjugat, das die Test-Flüssigkeit aufbereitet. Die Aufbereitung dauert eine festegelegte Zeitspanne von beispielsweise 4 Minuten, die abgewartet werden muss.

Nach dem Ende der Aufbereitung muss die aufbereitete Test-Flüssigkeit aus den Aufbereitungskammern 36, 37 auf ein Ende der Teststreifen des Analyseelements 39 gebracht werden. Dazu wird das Analyseelement 39 aus seiner in Fig.1 dargestellten Ruheposition in die in Fig. 2 dargestellte Anzeigeposition verschwenkt, in der die Hauptersteckungsrichtung 47 des Analyseelements 39 einen Winkel von 90° gegenüber der Axialrichtung 18 aufweist. Während des Verschwenkens des Analyseelements 39 durchstechen die Dorne 45, 46 die Folie 29, welche die Aufbereitungskammern 36, 37 in Richtung Analyseelement 39 abschliessen. Sobald die Folie 29 durchstochen ist, kann aufbereitete Test-Flüssigkeit von den Aufbereitungskammern 36, 37 auf ein Ende der Teststreifen des Analyseelements 39 gelangen. Die Test-Flüssigkeit fliesst dann wie oben beschrieben durch die Teststreifen, so dass nach einer festgelegten Wartezeit von beispielsweise 8 Minuten das Analyseergebnis an den Teststreifen abgelesen werden kann.

Gemäss Fig. 4 kann eine alternative Vorrichtung 110 zur Analyse einer Test-Flüssigkeit einen abnehmbaren Stopfen 158 aufweisen, der in das in Fig. 4 nicht sichtbare Eingangselement eingesteckt werden kann und sich damit an ein Betätigungselement 119 anschliesst. Der sonstige Aufbau und die Funktionsweise der Vorrichtung sind identisch mit denen der Vorrichtung 10 der Fig. 1 und 2.

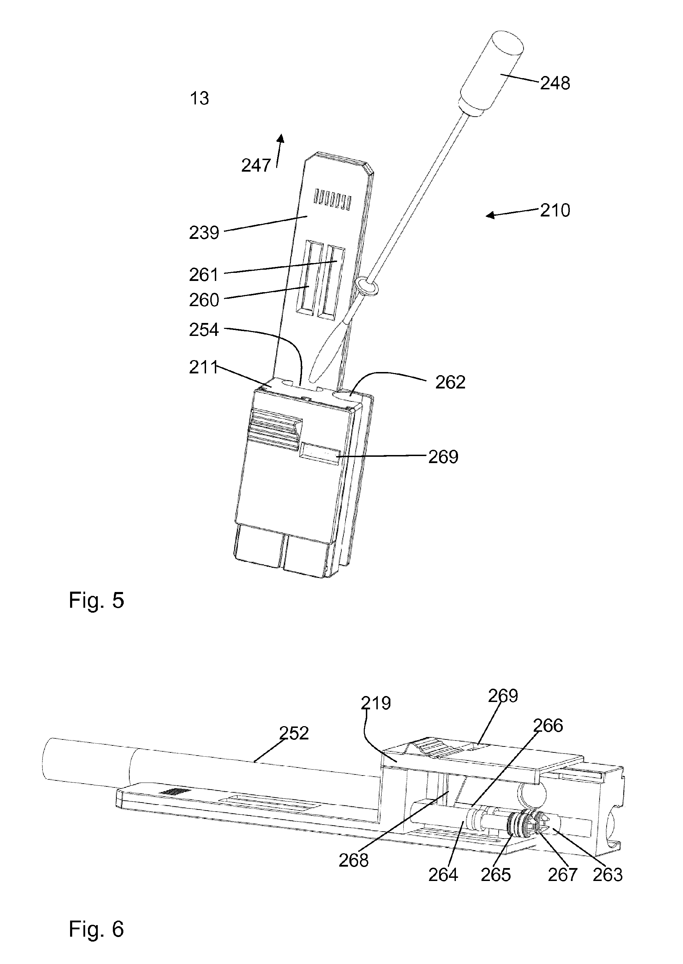

Gemäss Fig. 5 weist eine weitere alternative Vorrichtung 210 zur Analyse einer Test-Flüssigkeit ein Gehäuse 211 auf, das mit einem Analyseelement 239 verbunden ist. Das Analyseelement 239 weist zwei nicht näher dargestellte Teststreifen auf, die über zwei Ausnehmungen 260, 261 des Analyseelements 239 teilweise sichtbar sind. Die Vorrichtung 210 verfügt ebenfalls über einen Probensammler 248, der identisch mit dem Probensammler 48 aus Fig. 1 aufgebaut ist. Die Vorrichtung 210 weist einen parallel zu einer Haupterstreckungsrichtung 247 des Analyseelements 239 ausgerichteten Aufbewahrungsraum 262 auf, in welche der Probensammler 248 wie nur in Fig. 6 dargestellt inklusive einer Hülle 252 eingesteckt werden kann. Das Gehäuse 211 weist eine Eingangskammer 254 auf, welche nicht weiter dargestellte Auswaschflüssigkeit enthält und in die der Probensammler 248 eingesteckt und wie bereits beschrieben ausgewaschen werden kann.

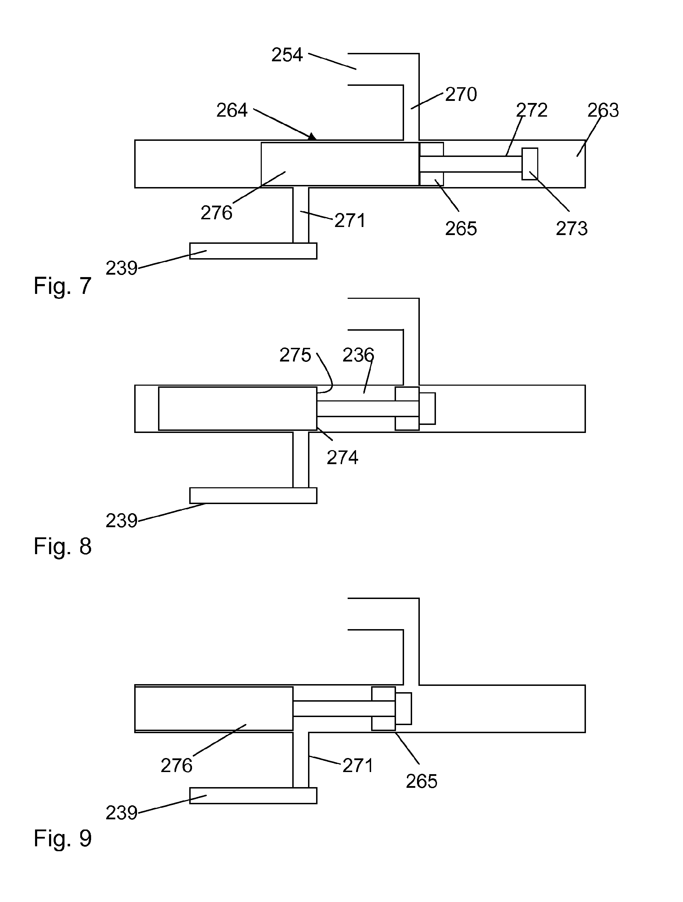

Wie in Fig. 6 dargestellt, verfügt das Gehäuse 211 über einen ersten zylinderförmigen Gehäuse-Innenraum 263 und einen zweiten nicht dargestellten weiteren zylinderförmigen Gehäuse-Innenraum, in welchen zwei Stössel 264, 266 und zwei Ringkolben 265, 267 angeordnet sind. Der Gehäuse-Innenraum 263 ist dabei parallel zur Haupterstreckungsrichtung 247 des Analyseelements 239 ausgerichtet. Die Funktionsweise der Stössels 264, 266 und der Ringkolbens 265, 267 wird im Zusammenhang mit den Fig. 7, 8 und 9 näher erläutert. Die Stössel 264, 266 können eine Ausgangsposition, eine Aufbereitungsposition und eine Analyseposition einnehmen. Zum Verschieben der Stössel 264, 266 sind die Stössel 264, 266 über einen Mitnehmer 268 mit einem gegenüber dem Gehäuse 211 in Haupterstreckungsrichtung 247 des Analyseelements 239 verschiebbares Betätigungselement 219 gekoppelt. Das Betätigungselement 219 weist eine Ausnehmung 269 auf, durch welche nicht dargestellte Markierungen auf dem Gehäuse 211, welche die aktuelle Position des der Stössel 264, 266 anzeigen, sichtbar sind. Die Anzeige der Position kann auch daraus bestehen, dass die Dauer einer bereits oben beschriebenen, bei der aktuellen Position der Stössel notwendigen Wartezeit angezeigt wird.

Die Funktionsweise der Vorrichtung 210 wird im Folgenden an Hand der Prinzipdarstellungen in Fig. 7, 8 und 9 näher erläutert, welche einen Schnitt durch den Stössel 264 und den Ringkolben 265 in Axialrichtung des Gehäuse-Innenraums 263 zeigen. Die Funktionsweise des Stössels 266 und des Ringkolbens 267 ist identisch.

Die Eingangskammer 254 ist über eine Zulaufleitung 270 mit dem Gehäuse-Innenraum 263 verbunden. Ausserdem führt eine Ablaufleitung 271 vom Gehäuse-Innenraum 263 zum Analyseelement 239.

Der Stössel 264 weist einen ersten Abschnitt 276 auf, der nur einen minimal kleineren Durchmesser als der Gehäuse-Innenraum 263 aufweist. An diesen ersten Abschnitt 276 schliesst sich ein zweiter Abschnitt 272 mit einem kleineren Durchmesser an, der von einem Mitnehmer 273 abgeschlossen wird, der wiederum einen etwas grösseren Durchmesser aufweist. Der Übergang vom ersten Abschnitt 276 auf den zweiten Abschnitt 272 erfolgt dabei an einem Absatz 274, der einen in Richtung Mitnehmer 273 orientierte Stirnfläche 275 aufweist. Auf dem zweiten Abschnitt 272 des Stössels 264 ist der Ringkolben 265 angeordnet, der damit zwischen dem Absatz 274 und dem Mitnehmer 273 auf dem Stössel 264 verschoben werden kann.

In der in Fig. 7 dargestellten Ausgangsposition des Stössels 264 ist der Stössel 264 so positioniert, dass der erste Abschnitt 276 des Stössels 264 sowohl die Zulaufleitung 270, als auch die Ablaufleitung 271 verschliesst. Damit kann keine Test-Flüssigkeit aus der Eingangskammer 254 in Richtung Gehäuse-Innenraum 263 fliessen. In dieser Position befindet sich der Stössel 264 im Ausgangszustand der Vorrichtung 210 und während dem Auswaschen des Probensammlers 248. Zur besseren Abdichtung kann der Stössel eine oder mehrere nicht dargestellte Dichtlippen oder Dichtelemente aufweisen.

Soll nun Test-Flüssigkeit aus der Eingangskammer 254 angesaugt werden, so wird der Stössel 264 mittels des in den Fig. 7, 8 und 9 nicht dargestellten Betätigungselements 219 bis in eine in Fig. 8 dargestellte Aufbereitungsposition verschoben. Bei dieser Verschiebung bleibt der verschieblich auf dem Stössel 264 angeordnete Ringkolben 265 zunächst stehen, so dass sich zwischen der dem Mitnehmer 273 und damit auch dem Ringkolben 265 zugewandten Stirnfläche 275 des genannten Absatzes 274 des Stössels 264 und dem Ringkolben 265 im Gehäuse-Innenraum 263 eine Aufbereitungskammer 236 ausgebildet, deren Volumen während der Verschiebung vergrössert wird. Der Ringkolben 265 bleibt so lange stehen, bis er vom Mitnehmer 273 erreicht und anschliessend mitgenommen wird. Ab diesem Zeitpunkt bleibt das Volumen der Aufbereitungskammer 236 unverändert. Die Aufbereitungsposition ist erreicht, wenn der Ringkolben 265 die Zulaufleitung 270 verschliesst. Um ein exaktes Einstellen der Aufbereitungsposition zu gewährleisten, ist zwischen Gehäuse 211 und Betätigungselement 219 eine nicht dargestellte Rastierung vorgesehen, die die das Betätigungselement 219 und damit den Stössel 264 in einzelnen Positionen fixiert, aber überdrückt werden kann. Zur besseren Abdichtung kann der Ringkolben eine oder mehrere nicht dargestellte Dichtlippen oder Dichtelemente aufweisen. Die genannte Rastierung kann auch innerhalb des Gehäuse-Innenraums angeordnet sein.

Durch das Verschieben des Stössels 264 von der Ausgangsposition in die Aufbereitungsposition wird das Volumen der Aufbereitungskammer 236 um ein definiertes Mass erhöht. Dadurch entsteht in der Aufbereitungskammer 236 ein Unterdruck, so dass eine definierte Menge an Test-Flüssigkeit über die Zulaufleitung 270 aus der Eingangskammer 254 angesaugt wird. Dort wird sie wie beschrieben aufbereitet.

Nach Abschluss der Aufbereitung wird die aufbereitete Test-Flüssigkeit über die Ablaufleitung 271 zu einem Ende des nicht weiter dargestellten Teststreifens des Analyseelements 239 geleitet. Dazu wird der Stössel 264 und mit ihm der Ringkolben 265 mittels des Betätigungselements 219 ausgehend von der Aufbereitungsposition in die Analyseposition verschoben. Während dieser Verschiebung gibt der erste Abschnitt 276 des Stössels 264 die Ablaufleitung 271 frei, so dass eine Verbindung zwischen der Aufbereitungskammer 236 und dem Analyseelement 239 besteht. Damit kann die aufbereitete Test-Flüssigkeit über die Ablaufleitung 271 zum Analyseelement 239 und damit durch den Teststreifen fliessen. Es ist auch möglich, dass zum Einstellen der Analyseposition der Stössel 264 noch weiter verschoben wird.

## Patentansprüche

1. Vorrichtung zur Analyse einer Test-Flüssigkeit mit
- einer Eingangskammer (54, 254),
- einer Aufbereitungskammer (36,37, 236) und
- einem Analyseelement (39, 239),
wobei
- die Eingangskammer (54, 254) dazu vorgesehen ist, die Test-Flüssigkeit aufzunehmen,
- Test-Flüssigkeit von der Eingangskammer (54, 254) über die Aufbereitungskammer (36, 37, 236) auf das Analyseelement (39, 239) bringbar ist und
- ein Volumen der Aufbereitungskammer (36, 37, 236) veränderbar ist,
**dadurch gekennzeichnet, dass**
das Volumen der Aufbereitungskammer (36, 37, 236) vergrösserbar ist und durch eine Vergrösserung des Volumens der Aufbereitungskammer (36, 37, 236) Test-Flüssigkeit aus der Eingangskammer (54, 254) in die Aufbereitungskammer (36, 37, 236) angesaugt wird.

2. Vorrichtung nach Anspruch 1,
**dadurch gekennzeichnet, dass**
in einem Ausgangszustand in der Eingangskammer (54, 254) eine Auswaschflüssigkeit angeordnet ist, welche gemischt mit einer Proben-Substanz die Test-Flüssigkeit ergibt.

3. Vorrichtung nach Anspruch 1 oder 2,
**gekennzeichnet durch**
- ein Gehäuse (11), welches einen Gehäuse-Innenraum (55) aufweist,
- ein Aufbereitungselement (20), welches die Aufbereitungskammer (36, 37) zumindest teilweise begrenzt und zumindest teilweise innerhalb des Gehäuse-Innenraums (55) angeordnet ist,
- ein Eingangselement (15), welches die Eingangskammer (54) zumindest teilweise begrenzt und zumindest teilweise innerhalb des Gehäuse-Innenraums (55) angeordnet ist,
wobei das Aufbereitungselement (20) und das Eingangselement (15) in einer Axialrichtung (18) hintereinander angeordnet und so miteinander verbunden sind, dass eine Verschiebung des Eingangselements (15) in Axialrichtung (18) das Volumen der Aufbereitungskammer (36, 37) vergrössert.

4. Vorrichtung nach Anspruch 3,
**dadurch gekennzeichnet, dass**
das Eingangselement (15) ein Betätigungselement (19, 119) aufweist, das ausserhalb des Gehäuses (11) angeordnet ist und das so ausgeführt ist, dass über das Betätigungselement (19, 119) eine Kraft zum Verschieben des Eingangselements (15) einleitbar ist.

5. Vorrichtung nach Anspruch 4,
**dadurch gekennzeichnet, dass**
das Eingangselement (15) ein erstes Gewinde (16) und das Gehäuse (11) ein korrespondierendes zweites Gewinde (17) aufweist, so dass bei einer Drehung des Eingangselements (15) gegenüber dem Gehäuse (11) das Eingangselement (15) in Axialrichtung (18) gegenüber dem Gehäuse (11) verschoben wird.

6. Vorrichtung nach einem der Ansprüche 3, 4 oder 5,
**dadurch gekennzeichnet, dass**
das Eingangselement (15) zur Vergrösserung des Volumens der Aufbereitungskammer (36, 37) gegenüber dem Gehäuse (11) von einer Ausgangsposition in eine Aufbereitungsposition verdreht werden kann und eine Verbindung zwischen der Eingangskammer (54) und der Aufbereitungskammer (36, 37) nur zwischen der Ausgangsposition und der Aufbereitungsposition besteht.

7. Vorrichtung nach einem der Ansprüche 3 bis 6,
**dadurch gekennzeichnet, dass**
zwischen der Aufbereitungskammer (36, 37) und dem Analyseelement (39) eine Folie (29) angeordnet ist, welche zum Starten der Analyse der Test-Flüssigkeit mittels eines Dorns (45, 46) durchstochen werden kann.

8. Vorrichtung nach Anspruch 7,
**dadurch gekennzeichnet, dass**
- das Analyseelement (39) den genannten Dorn (45, 46) aufweist,
- das Analyseelement (39) schwenkbar mit dem Gehäuse (11) verbunden ist und eine Ruheposition und eine Anzeigeposition einnehmen kann und
- das Analyseelement (29) so ausgeführt und angeordnet ist, dass bei einem Verschwenken gegenüber dem Gehäuse (11) der Dorn (45, 46) des Analyseelements (39) die zwischen Aufbereitungskammer (36, 37) und dem Analyseelement (39) angeordnet Folie (29) durchsticht und so die Analyse der Test-Flüssigkeit gestartet wird.

9. Vorrichtung nach Anspruch 8,
**dadurch gekennzeichnet, dass**
das Analyseelement (39) eine hauptsächlich quaderförmige Grundform aufweist und in der Ruheposition eine Haupterstreckungsrichtung (47) des Analyseelements (39) hauptsächlich parallel zur Axialrichtung (18) angeordnet ist.

10. Vorrichtung nach Anspruch 1 oder 2,
**gekennzeichnet durch**
- ein Gehäuse (211), welches die Eingangskammer (254), einen Gehäuse-Innenraum (263), eine Zulaufleitung (270) von der Eingangskammer (254) zum Gehäuse-Innenraum (263) und eine Ablaufleitung (271) vom Gehäuse-Innenraum (263) zum Analyseelement (239) aufweist,
- einen im Gehäuse-Innenraum (263) verschieblich angeordneten Stössel (264, 266), welcher einen Absatz (274) aufweist, und
- einen auf dem Stössel (264, 266) zumindest stückweise verschieblich angeordneten Ringkolben(265, 267),
wobei die Aufbereitungskammer (236) von einer dem Ringkolben (265) zugewandten Stirnfläche (275) des genannten Absatzes (274) des Stössels (264), dem Gehäuse-Innenraum (263) und dem Ringkolben (265) gebildet wird und
der Stössel (264) und der Ringkolben (265) so ausgeführt und angeordnet sind, dass bei einer Verschiebung des Stössels (264) von einer Ausgangsposition in eine Aufbereitungsposition der Ringkolben (265) zunächst bezogen auf den Gehäuse-Innenraum (263) stehen bleibt und so das Volumen der Aufbereitungskammer (236) vergrössert wird.

11. Vorrichtung nach Anspruch 10,
**dadurch gekennzeichnet, dass**
der Stössel (264) und der Ringkolben (265) so ausgeführt und angeordnet sind, dass in der Ausgangsposition die Zulaufleitung (270) von der Eingangskammer (254) zum Gehäuse-Innenraum (263) und/oder in der Aufbereitungsposition die Ablaufleitung (271) vom Gehäuse-Innenraum (263) zum Analyseelement (239) verschlossen sind.

12. Vorrichtung nach Anspruch 10 oder 11,
**dadurch gekennzeichnet, dass**
der Stössel (264) einen Mitnehmer (273) aufweist, welcher so ausgeführt ist, dass er eine Relativbewegung zwischen Stössel (264) und Ringkolben (265) begrenzt und der Stössel (264) und der Ringkolben (265)
- in der Aufbereitungsposition so angeordnet sind, dass die Zulaufleitung (270) von der Eingangskammer (254) zum Gehäuse-Innenraum (263) verschlossen ist und/oder
- eine Analyseposition einnehmen können, in welcher die Ablaufleitung (271) vom Gehäuse-Innenraum (263) zum Analyseelement (239) geöffnet und eine Verbindung von der Eingangskammer (254) zur Aufbereitungskammer (236) verschlossen ist.

13. Vorrichtung nach Anspruch 10, 11 oder 12,
**gekennzeichnet durch**
ein gegenüber dem Gehäuse (211) verschieblich angeordnetes und mit dem Stössel (264, 266) gekoppeltes Betätigungselement (219), mittels welchem eine Kraft zum Verschieben des Stössels (264, 266) eingeleitet werden kann.

14. Vorrichtung nach einem der Ansprüche 3 bis 13,
**dadurch gekennzeichnet, dass**
das Betätigungselement (219) eine Ausnehmung (269) aufweist, durch welche Markierungen, welche eine aktuelle Position des Eingangselements oder des Stössels (264, 266) anzeigen, sichtbar sind.

15. Vorrichtung nach einem der Ansprüche 3 bis 14,
**dadurch gekennzeichnet, dass**
das Gehäuse (11, 211) einen Aufbewahrungsraum (54, 262) für einen Probensammler (48, 248) aufweist.
